# EUROPEAN PATENT APPLICATION

(11) **EP 0 681 817 A1**
(43) Date of publication of application: **15.11.1995**
(21) Application number: 95918446.6
(22) Date of filing: 24.11.1994
(51) Int. Cl.: A61F 2/38

(54) **ARTICULAR PROSTHESIS OF THE PATELLA AND DISTAL FEMUR**

(30) Priority: 25.11.1993 ES 9302601
(71) Applicant: Pichel Moure, Carlos, Maria, E-36202 Vigo (ES)
(72) Inventor: Pichel Moure, Carlos, Maria, E-36202 Vigo (ES)
(86) International application number: ES9400125
(87) International publication number: WO9514445

(57) **Abstract**

Assembly of femoral (1) and patellar (2) components for a knee prosthesis, and of which the trochlear slopes (11), the femoral condyles (12) and the rotulian facets (22) have the same curvature and shape, preferably spherical; the femoral component has a transverse split or condilotrochlear groove between the trochlea and each of the condyles (14). The cinematic interaction between the design of the rotulian crest (21) and the trochlear throat (13) provides for a smooth displacement of the rotulian component from the trochlea to the condyles and vice versa without friction with the condilotrochlear groove (c) and maintains constantly a contact of the superficial type with the femoral component.

## Description

This invention is included in the field of artificial components for the arthroplastic substitution of joints and it specifically refers to a new set of femoral and patellar components for three compartmental prostheses for the knee.

In spite of numerous recent developments, the problems caused by the wearing down of the polyethylene, the loosening of the components and patellar complications continue to be the causes of the limited survival of knee prostheses which, in turn, reduce the potential benefits of this technique.

Although some devices of the "meniscal" type have managed to produce surface contact between the metal and the polyethylene of the femorotibial articulation, most of the femoral and patellar components currently available maintain single point or linear contact during their reciprocal performance. The small quantity of designs conceived to increase femoropatellar congruency only achieve surface contact at some stages of motion. On the other hand, all the technological solutions devised to substitute the femoropatellar joint have produced artificial components whose shape and performance very much differ from the normal morphology and the physiological behaviour of the natural joint, which is liable to cause kinematic conflict and increase the forces on the prosthetic components. The combination of these factors increases contact stress and makes the implant set be subject to abnormal forces which favour its wearing down and destruction.

The present invention intends to prolongue the survival of knee prostheses by means of working on the factors mentioned above. Therefore, objects of this invention are to perfect the geometry, to improve kinematics and increase the congruence of the femoropatellar articular components enabling permanent surface contact between the patellar polyethylene and the metallic femoral component within the total amplitude of its relative movement.

The location of the contact areas between the articular surfaces of the patella and femur during flexion and extension movements of the natural knee responds to a characteristic pattern. An important aspect of this pattern is that when flexing the knee, certain areas of the articular surface of the patellar facets make successive contact with areas of the trochlea at first and with regions of the most frontal part of the femoral condyles later, carrying out the reverse trajectory when extending the knee.

To reproduce this behaviour and achieve area contact at all times between the patellar polyethylene and the metallic femoral component, the patellar facets have to be completely congruent with the trochlear sides and with the portions of the femoral condyles with which they articulate. To obtain this degree of congruency in this invention, each trochlear side and the corresponding femoral condyle - or at least its frontal part - have the design of two sectors of independent solid spheres of the same curvature and the ipsilateral patellar facet has the design of a sector of a hollow sphere of the same radius. A similar effect can be obtained with non-spherical surfaces provided their sagital and cross sections are circular and the cross section radius is constant.

Thus, by preference, all of the described surfaces (condyles, trochlear sides and patellar facets) that correspond to each of the sagital halves of the femoral and patellar components have the shape of sectors of spheres of the same radius; although the radii that correspond to each half may or may not be equal and the hind part of the femoral condyles destined to solely articulate with the tibia may be built with a different sagital radius from that corresponding to its frontal part destined to also articulate with the patella. The fact that the medial and lateral radii can be similar or dissimilar enables the manufacture of components with symmetrical or asymmetrical articular surfaces. On the other hand, manufacturing completely spherical condyles or, on the contrary, modifying the radius of its most hind part will depend on the type of tibial polyethylene selected to join with the femoral component presented in this invention.

As a consequence of this geometric construction of the femoral component, between each trochlear side and the ipsilateral femoral condyle - formed by the union of two sectors of spheres with different centres - a crosswise sulcus appears which is similar to the anatomical condylotrochlear sulcus of the natural femur.

The trochlear sides of the femoral component come together at a central channel or trochlear groove whose distal and posterior end terminates in a sagital cleft that separates both femoral condyles or intercondylear notch. The cross section of the groove has the shape of an arc of a circumference of constant radius and its sagital sections have the shape of curves made up by three arcs of circumference (proximal, intermediate and distal respectively) tangent at their points of union. In the case of the sagital sections that delimit the groove transversely, the centre and radius of the intermediate arc coincide with the centre and radius that correspond to the adjacent section of the trochlear groove, while the distal arc forms part of a circumference tangent to said adjacent section and to the section of the condyle sphere contained in the same plane, both being circular and of the same radius, although the condyle section can be materially nonexistent due to the presence of the intercondylear notch. Preferably, the proximal arc is given the same radius as the distal arc.

The lateral facets of the patellar component come together at a central crest which is perfectly congruent with the trochlear groove in a medial-lateral direction since both have a circular section of the same radius in the transversal plane. In the sagital direction, the patellar crest is concave and has only one curvature radius, which coincides with that of the distal longitudinal arc of the trochlear groove.

According to the described arrangement, the patellar crest rests along all its length on the proximal part of the trochlear groove and, shortly after the flexion movement has been initiated, the sliding of the patellar component is directed by the spherical contact of the patellar facets upon the trochlear sides. However, from the point where the intermediate and distal arcs of the trochlear groove are tangent onwards, the movement is directed by the patellar crest as it slides over the distal arc of the trochlear groove; which, owing to its geometric design, compels the patellar facets to separate from the trochlear sides to make contact again with the femoral condyles once the distal point of tangency has been overcome, point that can be materially nonexistent due to the presence of the intercondylear notch.

This way the condylotrochlear sulcus is overcome and the succession of femoropatellar areas of contact of the natural knee is simulated which contributes to reduce the magnitude of the forces transmitted by the joint. Moreover, the patellar component and the femoral component sustain contact on a significant area of their articular surfaces throughout the total range of their relative movement, avoiding linear or point contact produced in most of the previous designs. The contact surfaces of the articulation of the femoral and meniscal components can also be enlarged and allow total congruency between them on a permanent basis if desired, as opposed to other current designs that are forced to reduce meniscalfemoral congruency to obtain greater femoropatellar surface contact.

The principles stated in the preceeding explanation and a graphic model are represented in the following figures:
Figure 1 is a sagital section of the femoral component which coincides with one of the planes which delimit the trochlear groove transversely.
Figure 2 is the patellar component in a lateral direction.
Figure 3 is a sagital section of the femoral component which illustrates the design of the trochlear groove.
Figure 4 is a diagram that represents how the reciprocal performance of the components compels the patellar facets to separate from the trochlear sides before reaching the condylotrochlear sulcus and to make contact again with the condyles once that gap is overcome.
Figure 5 represents a frontal view of the femoral component.
Figure 6 is a lower part view of the femoral component.
Figure 7 is a dorsal view of the patellar component.
Figure 8 is an axial view of the patellar component.

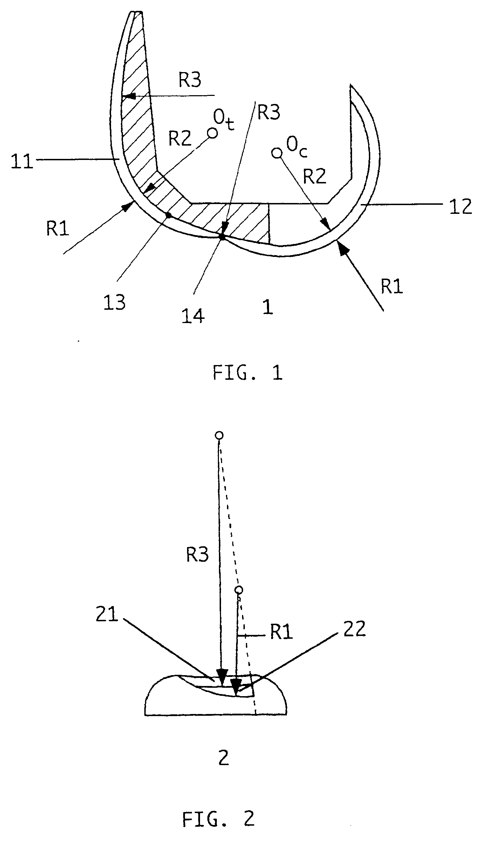

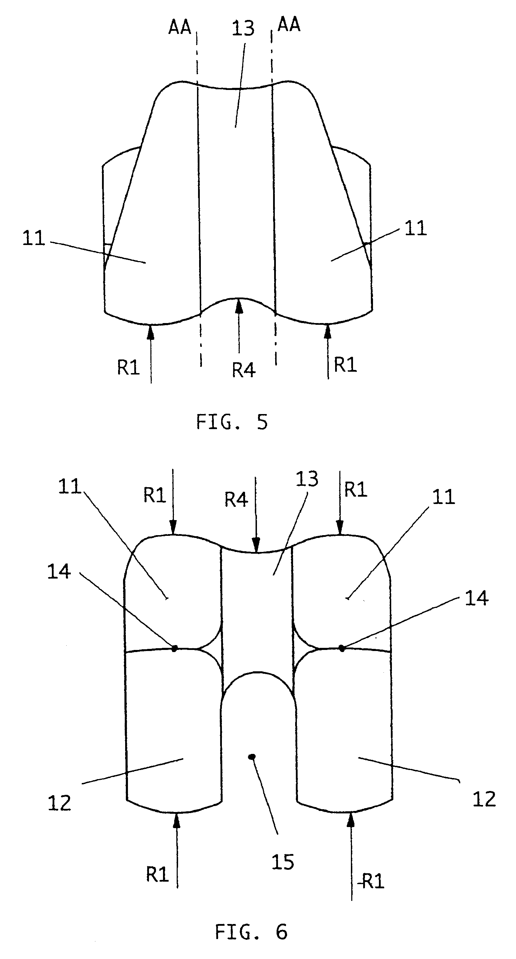

With reference to figures 1, 3, 5 and 6 , the femoral component (1), made of a metallic alloy, has five parts, two of which correspond to the medial and lateral trochlear sides (11), another two to the medial and lateral femoral condyles (12), and the last one to the trochlear groove (13).

Parts 11 and 12 have the shape of sectors of spheres of the same radius (R₁), although the common radius of the medial trochlear and medial condylear sectors can differ slightly from that corresponding to the lateral sectors. The union of the curvatures of each trochlear side with the curvature of the ipsilateral condyle forms a cleft or condylotrochlear sulcus (14).

The trochlear groove (13) unites the medial and lateral parts of the component and ends in an intercondylear notch (15) beyond the condylotrochlear sulcus. This notch separates the lower and rear parts of the condyles (12). In a transversal direction, its shape is concave and only has one radius (R4). In a sagital direction, it is convex and has two radii (R₂ and R₃) and at least two curvature centres (Og and Ot).

In the sections that correspond to the planes (AA) that delimit the trochlear groove in a medial-lateral direction, the radius of its distal portion or arc (18) corresponds to the radius of a common tangent circumference to the adjacent sections of the corresponding trochlear or condylear spheres which have the shape of circles with centres Ot and Og respectively and radius (R₂) that coincides with the radius of the intermediate arc of said groove. The proximal arc (16) can be designed with the same radius (R₃) as the distal arc.

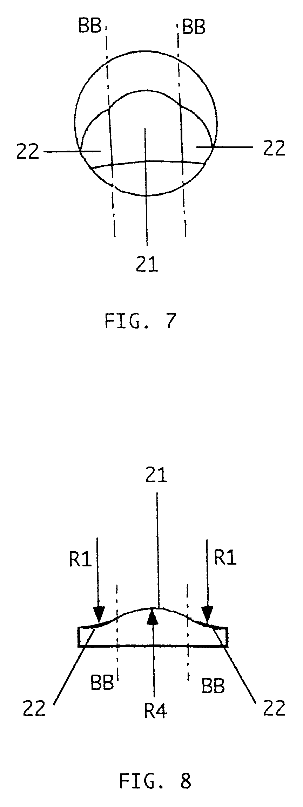

Concerning figures 2,7 and 8, the patellar component (2), made of high density polyethylene, has a rounded shape in the frontal plane, with a prominence or patellar crest (21) on the central part of its articulating surface. This crest is concave in a sagital direction and convex in a crosswise direction. The radius of the sections that delimit it in a transversal direction (BB) being the same as radius R₃ of the femoral component, while the radius of the cross section is equal to radius R₄ of the trochlear groove (13). At both sides of this crest there are two concave basins or patellar facets (22) with radius R₁, which have the same centre as the respective spherical trochlear and condylear sectors of the femoral component.

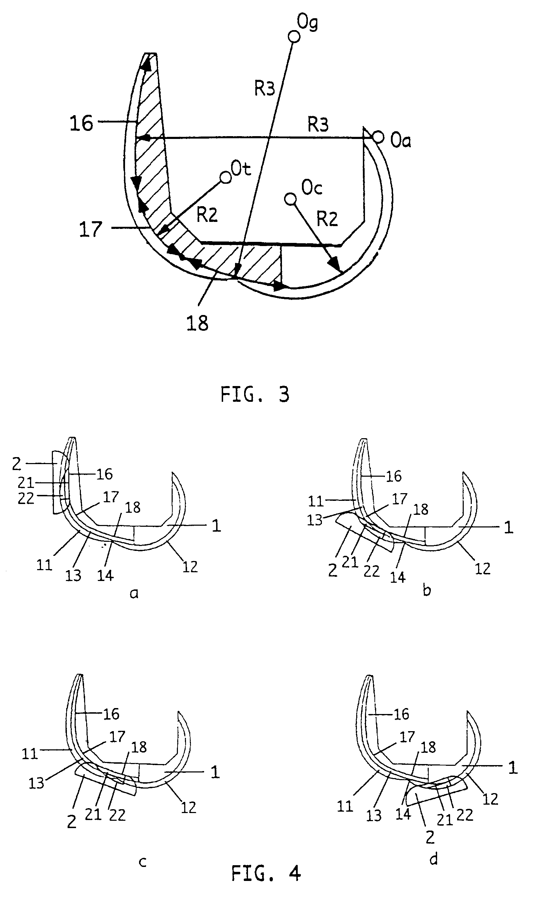

As a consequence of this geometric set up, as is illustrated in figure 4, in the first degrees of the flexion movement of the knee (a) the patellar crest (21) makes contact with the higher part of the trochlear groove (16). Later (b) main contact is made between the concentric surfaces of the patellar facets (22) and the trochlear sides (11). From the second change of the radius of the trochlear groove (c) onwards, contact is transferred to the patellar crest (21) as a consequence of its complete congruency with the distal arc of the groove (18), this causes the separation of the patellar facets (22) from the trochlear sides (11). Once the contact of the patellar crest with the trochlear groove (d) is over, when the patella reaches the intercondylear notch, the patellar facets make new contact with the femoral condyles. A "patellar flight" is produced in this way which prevents the patellar facets from blocking at the condylotrochlear sulcus (14) and the succession of points of contact in the natural knee is simulated.

## Claims

1. A set comprising a knee prosthesis femoral component and patellar component designed in a way so that the medial and lateral facets of the patellar component are completely congruent with the corresponding articular surfaces of the trochlear sides and the condyles of the femoral component.

2. A set according to claim number 1 whose reciprocal performance simulates the succession of points of contact in the natural knee, allowing the patellar facets to establish successive contact with the trochlear sides and the femoral condyles throughout the flexion of the knee and to carry out the reverse trajectory during the extension of the knee.

3. A femoral component according to claims numbers 1 and 2 with the marked feature that the articular surfaces of both trochlear sides and the articular surfaces of the femoral condyles, by preference, have the shape of sectors of spherical surfaces, arranged in such a way so that a condylotrochlear sulcus is formed between each trochlear side and the ipsilateral condyle.

4. A femoral component according to claim number 3 wherein the four spherical sectors, two trochlear and two condylear, have the same radius; with the possibility that the radius of the medial sectors may be slightly different from the radius of the lateral sectors.

5. A femoral component according to claims numbers 3 and 4 whose condylear surfaces are separated at their central part by a notch.

6. A femoral component according to claims numbers 3 and 5 wherein the trochlear sides come towards a groove or central sulcus whose cross section, which is concave, has the shape of an arc of a circumference and whose longitudinal section, which is convex, has at least two curvature radii.

7. A femoral component according to claims numbers 3 and 6 wherein the centres and radii of the two most peripheral longitudinal sections of the trochlear groove coincide at their proximal or intermediate part with those of the adjoining sections of both trochlear sides, while at their distal part they coincide with those of two circumferences that are common tangent respectively to each of the mentioned trochlear sections and to the condylear section ideally contained in the same plane.

8. A patellar component according to claims numbers 1 and 2 characterised because its medial and lateral articular facets by preference have the shape of two sectors of hollow spheres.

9. A patellar component according to claim number 8 wherein the sectors of hollow spheres that form its facets have the same radius as the trochlear and condylear sectors of solid spheres with which they articulate.

10. A patellar component according to claims numbers 8 and 9 whose medial and lateral spherical facets are separated by a central crest which is craneocaudally orientated for which both its cross section, which is convex, and its longitudinal section, which is cocave, have the shape of arcs of circumference.

11. A patellar component according to claims numbers 8, 9 and 10 wherein the radius of the cross sections of the central crest coincides with the transversal radius of the trochlear groove, while the single radius of each of its most peripheral longitudinal sections coincides with that of the common tangent circumferences mentioned in claim 7.

12. A set of components according to any of the preceeding claims wherein the design of its central crest forces the medial and lateral parts of the patellar component to separate from the femoral component when approaching the condylotrochlear sulcus and to come into contact again once that sulcus has been overcome.

13. A set of components according to any of the preceeding claims wherein the geometry of the nonfunctional areas of the intraarticular surfaces, that is, the regions of the components that will not make area contact during their relative movement, can be designed freely regarding objectives of kinematics optimization, structural resistance or morphological suitability.

14. A set of components according to any of the preceeding claims wherein, with the aim to obtain different models or custom made prostheses, each corresponding patellar facet, trochlear side and frontal part of the femoral condyle can be built with non-spherical congruent surfaces; the hind part of the condyles can have a different sagital radius from its frontal part and the medial and lateral halves of both components can be asymmetric.
